(19) Europäisches Patentamt
European Patent Office
Office européen des brevets

(11) Publication number: 0 063 359 B1

(12) **EUROPEAN PATENT SPECIFICATION**

(45) Date of publication of patent specification: **11.12.85**

(51) Int. Cl.[4]: **C 07 D 211/90**, A 61 K 31/445 // C07C69/72, C07C79/46, C07C101/34

(21) Application number: **82103145.7**

(22) Date of filing: **14.04.82**

(54) **1,4-Dihydropyridine derivatives and processes for preparing the same.**

(30) Priority: **18.04.81 JP 57855/81**

(43) Date of publication of application: **27.10.82 Bulletin 82/43**

(45) Publication of the grant of the patent: **11.12.85 Bulletin 85/50**

(84) Designated Contracting States: **BE CH DE FR GB IT LI LU NL SE**

(56) References cited:
**DE-A-2 629 892**
**DE-A-2 756 226**
**FR-A-2 435 471**
**FR-A-2 438 654**

**DERWENT JAPANESE PATENTS, Section Chemical, Derwent publications Ltd., vol. 4, no. 98 (C-18)(580), 15th July 1980, page 150;**

(73) Proprietor: **BANYU PHARMACEUTICAL CO., LTD.**
**7-8, Nihonbashi Honcho 2-chome**
**Chuo-ku Tokyo 103 (JP)**

(72) Inventor: **Miyano, Tetsuji**
**1-26, Shinonolaze Narumi-cho Midori-ku**
**Nagoya-shi Aichi-ken (JP)**
Inventor: **Suzuki, Kunio**
**4-2, Ohgida-machi Minami-ku**
**Nagoya-shi Aichi-ken (JP)**
Inventor: **Harada, Nobuo**
**61-4, Ohike Hane-cho**
**Okazaki-shi Aichi-ken (JP)**
Inventor: **Asai, Akira**
**No. 23, Kamiarako Hashira-cho**
**Okazaki-shi Aichi-ken (JP)**

(74) Representative: **Wächtershäuser, Günter, Dr.**
**Tal 29**
**D-8000 München 2 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 063 359 B1

Courier Press, Leamington Spa, England.

**Description**

BACKGROUND OF THE INVENTION:

FIELD OF THE INVENTION:

This invention relates to novel 1,4-dihydropyridine derivatives or the pharmacologically acceptable salts thereof and processes for their preparation. The compounds of the present invention possess vasodilation activities and hypotensive activities, and are expected to be highly useful medicines for the blood circulatory system.

DESCRIPTION OF THE PRIOR ARTS:

As compounds which have so far been known for such use, there are, for example, 1,4-dihydropyridine derivatives to be enumerated in the following.

S-1: nifedipine; 4-(o-nitrophenyl)-2,6-dimethyl-1,4-dihydropyridine-3,5-dicarboxylic acid dimethyl ester (cf. U.S. Patent No. 3,485,847);

S-2: 4-(m-nitrophenyl)-2,6-dimethyl-1,4-dihydropyridine-3,5-dicarboxylic acid bis-(β-propoxyethyl-ester) (cf. Japanese Patent Publication No. 37,349/71);

S-3: 4-(m-nitrophenyl)-2-hydroxymethyl-6-methyl-1,4-dihydropyridine-3,5-dicarboxylic acid diethylester (cf. Japanese Laid-Open Patent Application No. 5777/77).

Other related compounds are known from DE—A—2756226, DE—A—2629892 and FR—A—2438654.

SUMMARY OF THE INVENTION:

As the result of diligent research activities done by the present inventors, novel 1,4-dihydropyridine derivatives have been discovered and thus the present invention completed. These novel compounds have their structural characteristics in the substituent group at No. 2 and No. 3 positions of 1,4-dihydropyridine ring, possess much stronger vasodilation activities than the abovementioned known compounds, and are weaker in toxicity, hence are useful as medicines.

It is therefore an object of the present invention to provide novel 1,4-dihydropyridine derivatives or the pharmacologically acceptable salts thereof having improved vasodilation activities and hypotensive activities, and particularly suitable as medicines for blood circulatory system.

It is another object of the present invention to provide processes for preparing the abovementioned compounds.

Thus, the present invention provides novel 1,4-dihydropyridine derivatives represented by the following general formula (I) and the pharmacologically acceptable salts thereof.

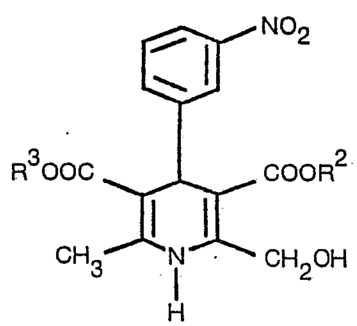

(I)

where $R^2$ represents β-propoxyethyl; $R^3$ denotes β-propoxyethyl, β-allyloxyethyl or β-(N-methyl-N-benzylamino)ethyl.

The compounds (I) according to the present invention can be prepared by the following various processes.

(a) A process, wherein a compound represented by the following general formula (VI) is prepared by reacting a compound represented by the following general formula (II) with an amine compound represented by the following general formula (III), or by reacting a compound represented by the following general formula (IV) with an amine a compound represented by the following general formula (V):

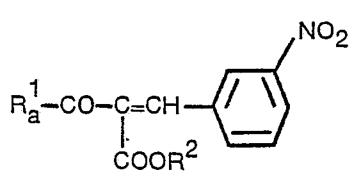

(II)

wherein $R_a^1$ represents dialkoxymethyl and $R^2$ is β-propoxyethyl

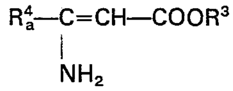

(III)

where $R^3$ represents β-propoxyethyl, β-allyloxyethyl or β-(N-methyl-N-benzylamino)ethyl; and $R^4_a$ denotes methyl;

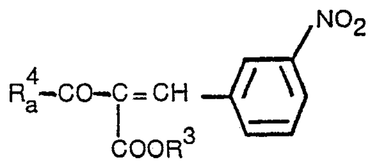

(IV)

where $R^3$ and $R^4_a$ are as defined in the preceding general formulas (II) and (III);

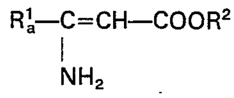

(V)

where $R^1_a$ and $R^2$ are as defined in the preceding general formula (II):

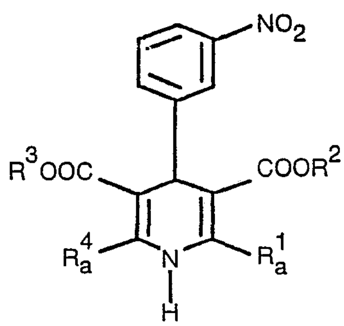

(VI)

where $R^1_a$, $R^2$, $R^3$, $R^4_a$ are as defined in the preceding general formulas (II) or (V)

(b) A process, wherein a compound represented by the following general formula (VII) is prepared by hydrolysis of the compound of the general formula (VI):

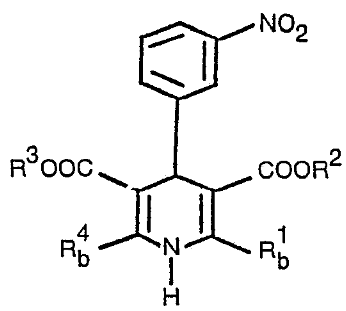

(VII)

where $R^1_b$ represents formyl; $R^4_b$ donates methyl; and $R^2$ and $R^3$ are as defined above.

(c) A process, wherein a compound represented by the following general formula (VIII) is prepared by reduction of the compound of the general formula (VII):

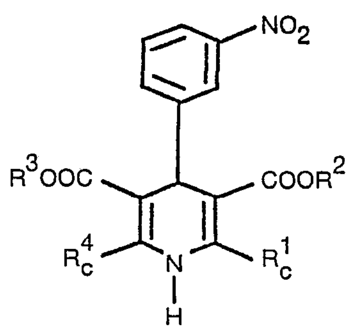

(VIII)

where $R^1_c$ denotes hydroxymethyl; $R^4_c$ represents methyl; and $R^2$ and $R^3$ are as defined above.

(d) A process, wherein a compound represented by the following general formula (XI) is prepared by reacting a compound represented by the following general formula (IX) with methylbenzylamine

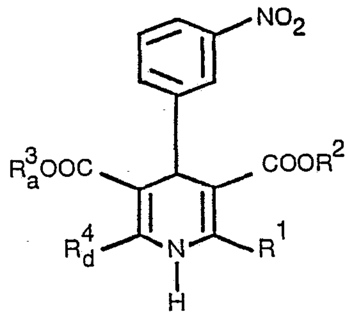

(IX)

where $R^1$ is hydroxymethyl;

$R^3_a$ represents haloethyl; $R^4_d$ denotes methyl; and $R^2$ is as defined above;

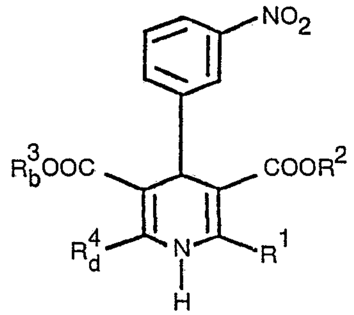

(XI)

wherein $R^3_b$ is β-(N-methyl-N-benzylamine)ethyl; and $R^1$, $R^2$ and $R^4_d$ are as defined above.

The foregoing objects, the structural characteristics of the compounds according to the present invention as well as the process of preparing the same will become more apparent and understandable from the following detailed description thereof along with several preferred examples for preparing the same.

DETAILED DESCRIPTION OF THE PREFERRED EMBODIMENTS:

The compound (I) according to the present invention can be prepared by the various methods mentioned above.

For the dialkoxymethyl contained in $R^1_a$ in the abovementioned general formula, there may be enumerated dimethoxymethyl, diethoxymethyl and dipropoxymethyl. $R^1_c$ may be hydroxymethyl, $R^1_h$ and $R^4_b$ also contain formyl. For the haloethyl and N,N-di-substituted aminoalkyl contained in $R^3_a$ and $R^3_b$, there may be enumerated those same groups contained in $R^3$ as already explained in the foregoing. For the haloethyl there are enumerated β-chloroethyl and β-bromoethyl. Alkyl contained in $R^4_a$ and $R^4_d$, is methyl. $R^2$ and $R^3$ are the same groups as already mentioned in the foregoing.

The raw material compounds (II, III, IV, V) for use in the present invention, regardless of whether they have been well known, or do not even appear in any published literature, can be synthesized by known methods. The raw materials (II) and (V) are novel compounds not appearing in the literature. As to the compounds (III) and (IV), those having alkyl removed from $R^3$ and $R^4_a$ are as novel as the compounds (II) and (V). These compounds can be synthesized by the reactions shown in the following.

(1) The raw material compound (II) is synthesized by reacting an ester of 4,4-dialkoxyalkyl acetoacetic acid (II-a) with an aldehyde (II-b).

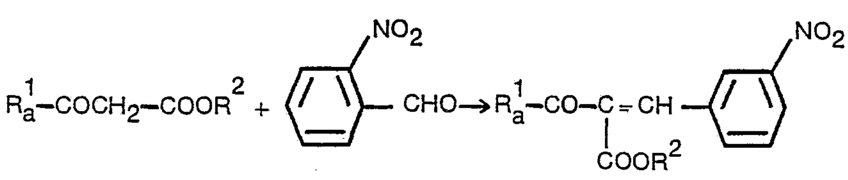

(II–a) (II–b) (II)

where $R^1_a$ and $R^2$ are as defined above.

(2) The raw material compound (V) is synthesized by reacting an ester of 4,4-dialkoxyalkyl acetoacetic acid (II-a) with ammonia or its salts.

$$R^1_a\text{—}COCH_2\text{—}COOR^2 + NH_3 \rightarrow R^1_a\text{—}\underset{\displaystyle NH_2}{\underset{|}{C}}=CH\text{—}COOR^2$$

(II-a) (V)

wherein $R^1_a$ and $R^2$ are as defined above.

In the following, detailed explanations will be given as to the reactions to take place at the time of preparing the compounds (I) of the present invention.

The abovementioned process (a) is concerned with the synthesizing method of the compound (VI) by reacting an ester compound of 4-(substituted, or non-substituted) 2-substituted benzylidene acetoacetic acid (II or IV) with amine compounds (III or V). This reaction is conducted usually at room temperature or under heat. For the reaction solvent, there may be used methanol, ethanol, propanol, isopropanol, butanol, benzene, or toluene. In some case, the solvent needs not be used particularly. In this reaction, if the base such as, for example, pyridine or sodium carbonate is used, the reaction as a whole is advantageously accelerated. The reaction time ranges from 2 to 20 hours, and it is usually from 6 to 10 hours.

The abovementioned process (b) is concerned with the synthesizing method of oxo compound (compound VII) by hydrolysis of the compound (VI). The hydrolytic reaction is carried out in the presence of an acid such as hydrochloric acid, sulfuric acid, p-toluene or sulfonic acid. The acid is used in an equivalent mol or in an excessive amount with respect to the compound (VI). This reaction is usually carried out in a cooling or warming condition. For the reaction solvent, there may be used acetone, dioxane, alcohol or N,N-dimethylformamide. The reaction time ranges from 0.5 to 5 hours.

The abovementioned process (c) relates to the synthesizing method of 1,4-dihydropyridine derivative (VII), wherein the second and/or sixth position of oxo compound (compound (VII)) is substituted for hydroxyalkyl by its reduction. For the reducing agent in this reaction, there may be used sodium borohydride, potassium borohydride, lithium borohydride, or other alkali metal borohydrides. The reaction is conducted in a cooling or warming condition. The reaction solvent to be used are: methanol, ethanol, propanol, isopropanol or dimethylformamide. The reaction time ranges from 0.5 to 5 hours.

The abovementioned process (d) relates to the synthesizing method of the compound (XI) by reacting haloalkyl ester derivative (compound IX) with the amine compound. The amine compound to be used for this reaction is N-methyl-N-benzyl amine. For the reaction solvent, there may be used methanol, ethanol, propanole, isopropanole, N,N-dimethylformamide or tetrahydrofuran. The reaction should preferably be carried out in the presence of lithium iodide or sodium iodide. The reaction is effected at a room temperature or under heat for 2 to 20 hours.

The compound (I) as the end product which has been synthesized by each of the above-described processes can be refined, isolated, and collected by the ordinary operations such as the extraction process using an organic solvent, the column chromatographic process using silica gel or alumina, and crystallizing process, and others.

In the case of the compounds having the salt-forming capability, they may be made into their salts with an inorganic acid such as hydrochloric acid or an organic acid such as citric acid.

The compounds (I) according to the present invention possess vasodilation activities and hypotensive activities. In particular, since they have strong coronary vasodilation activities and weak toxicity, the compounds find their expected use for cure of diseases in the circulatory system such as hypertension, cardiac insufficiency, angina pectoris, myocardial infarction, and brain vascular trouble.

The following are the test results for pharmacological effects and toxicity of those compounds (B to D) belonging to the compound (I) provided by the present invention, as compared with nifedipine (S-1) which is a known compound, or with comparative compounds A and E.

1. Tested compounds:

S-1: 4-(o-nitrophenyl)-2, 6-dimethyl-1, 4-dihydropyridine-3, 5-dicarboxylic acid dimethyl ester (nifedipine)

A: 4-(m-nitrophenyl)-2-hydroxymethyl-6-methyl-1,4-dihydropyridine-3, 5-dicarboxylic acid 3-β-propoxyethyl ester-5-ethyl ester

B: 4-(m-nitrophenyl)-2-hydroxymethyl-6-methyl-1,4-dihydropyridine-3, 5-dicarboxylic acid 3-β-propoxyethyl ester-5-β-allyloxyethylester

C: 4-(m-nitrophenyl)-2-hydroxymethyl-6-methyl-1, 4-dihydropyridine-3, 5-dicarboxylic acid bis(β-propoxyethyl ester)

D: 4-(m-nitrophenyl)-2-hydroxymethyl-6-methyl-1, 4-dihydropyridine-3, 5-dicarboxylic acid 3-β-propoxyethyl ester-5-β-(N-methyl-N-benzyl amino) ethyl ester hydrochloric acid salt

E: 4-(m-nitrophenyl)-2, 6-bis(hydroxymethyl)-1, 4-dihydropyridine-3, 5-dicarboxylic acid 3-ethyl ester-5-β-propoxyethyl ester

2. Test results:

The coronary vasodilation effects of the compounds in rabbits and the acute toxicity in mice according to the Langendorff's method* are as shown in the following Table.

(*) O. Langendorff: Pflügers Arch. Physiol., *61*, 291-322 (1895)

TABLE

Test Results on Medicinal Effect and Toxicity

| Compound Tested | Coronary Vasodilation ICD$_{50}$ (g/ml) | Acute Toxicity LD$_{50}$ (mg/kg), i.v. |
|---|---|---|
| Comparative Compound: (S—1) | $2.2 \times 10^{-7}$ | 115 |
| Compound: A (Comp.) | $2.5 \times 10^{-7}$ | 119 |
| ,, : B | $1.8 \times 10^{-7}$ | 103 |
| ,, : C | $2.0 \times 10^{-7}$ | 129 |
| ,, : D | $1.5 \times 10^{-7}$ | 168 |
| ,, : E (Comp.) | $3.2 \times 10^{-7}$ | 127 |

3. Test methods:

a) Coronary Vasodilation:

In acccordance with the Langendorff's method, the dosage effect of these compounds in the coronary arteries of a sample of the heart of rabbit as taken out of its body was found out. Strength of the coronary vasodilation was evaluated by a sample dosage (ICD$_{50}$ (g/ml)) which increased the blood flow rate in the coronary arteries by 50%.

b) Acute Toxicity:

Using DM-type male mice (18 to 22 g.), the LD$_{50}$ values were calculated by the up-and-down method in the intravenous dosage.

Since the compounds (I) according to the present invention are expected to be useful as vasodilators as well as hypotensive drugs, the mode of administration of the compound for that purpose may be chosen from any of the following: parenteral administration such as intravenous injection, hypodermic or intramuscular injection, or rectum administration; or oral administration in a form of tablets, powders, granules, capsules, sublingual tablets, or syrups. The quantity of administration of the compound depends upon condition of disease, age, and weight of the patient, and the mode of its administration to him. The dosage is usually from 0.1 to 1,000 mg per day for an adult, or preferably from 1 to 100 mg. The abovementioned medicinal compounds can be prepared by the well known methods.

PREFERRED EXAMPLES:

With a view to enabling those skilled in the art to readily practise the present invention, several preferred embodiments of the processes for preparing the novel compounds according to the present invention are given in the following, together with the processes for preparing the raw material compounds by the known methods.

*Preparation of Raw Material Compounds*

Reference Example 1

4,4-Dimethoxy acetoacetic acid methyl ester (25 g.), 2-propoxyethanol (150 ml), and molecular sieve 4A (50 g.) were mixed, and subjected to reaction for 12 hours under agitation. After filtration of the reaction mixture, the filtrate was concentrated under a reduced pressure, and the residue was distilled under the vacuum to obtain 4,4-dimethoxy acetoacetic acid β-propoxyethyl ester (25 g.) in colorless liquid.

IR (Liquid film): 2950, 1750, 1730, 1660, 1445, 1315, 1220, 1100, 1070, 995 cm$^{-1}$.

NMR (90MHz, in $CDCl_3$): δ0.9 (t, J=7Hz, 3H), 1.6 (m, 2H), 3.45 (m, 8H), 3.65 (s, 2H), 3.72 (t, J=4—5Hz, 2H), 4.33 (t, J=4—5Hz, 2H), 4.65 (s, 1H)

The thus obtained 4,4-dimethoxy acetoacetic acid β-propoxyethyl ester (24.8 g.) was mixed with m-nitrobenzaldehyde (15.1 g.), piperidine (0.5 ml), acetic anhydride (0.5 ml), and benzene (50 ml), and refluxed under heat for four hours under the azeotropic dehydration condition. After cooling, the reaction mixture

was washed with water, and dried, after which the solvent was fractionated by the distillation under a reduced pressure. Oily substance in the residue was separated and refined in a silica gel column chromatography using a developing solvent (hexane:ethyl acetate = 3:1), thereby obtaining 2-(m-nitrobenzylidene)-4,4-dimethoxy-acetoacetic acid β-propoxyethyl ester (24 g.) in the form of light yellow oily substance.

UV (in MeOH): $\lambda_{max}$ = 266 nm.

IR (Liquid film): 2950, 2880, 1720, 1610, 1530, 1450, 1350, 1250, 1200, 1100, 985, 905, 810, 735, 680 $cm^{-1}$.

NMR (90 MHz, in $CDCl_3$): δ0.93 (t, J=8Hz, 3H), 1.58 (m, 2H), 3.35 (t, J=8Hz, 2H), 3.45 (s, 6H), 3.68 (t, J=5Hz, 2H), 4.42 (t, J=5Hz, 2H), 4.96—5.12 (a pair of singlet, 1H), 7.4—8.4 (m, 5H).

Reference Example 2

4,4-Dimethoxy acetoacetic acid β-propoxyethyl ester (24.8 g.) obtained by the process as described in the first half of the reference example 1 was dissolved in 2-propoxy ethanol (20 ml), into which solution ammonia gas was blown for four hours. After the reaction mixture was concentrated under a reduced pressure to fractionate ammonia, it was further subjected to distillation under the vacuum, whereby 4,4-dimethoxy-3-amino crotonic acid β-propoxyethyl ester (23 g.) was obtained.

bp: 153—165°C/2mm Hg

IR (Liquid film): 3460, 3350, 2950, 1670, 1620, 1560, 1445, 1365, 1345, 1270, 1165, 1000, 790 $cm^{-1}$.

NMR (90 MHz, in $CDCl_3$): δ0.9 (t, J=7Hz, 3H), 1.58 (m, 2H), 3.35 (t, J=7Hz, 2H), 3.45 (s, 6H), 3.65 (t, J=5Hz, 2H), 4.4 (t, J=5Hz, 2H), 4.53 (s, 1H), 4.65 (s, 1H).

Comparative Example 1

(a) 2-(m-Nitrobenzylidene)-4,4-dimethoxy acetoacetic acid β-propoxyethyl ester (4 g.) and 3-amino crotonic acid ethyl ester (1.3 g.) were dissolved in ethanol (30 ml), and reacted for 8 hours under agitation at a temperature of 80 to 90°C. The reaction mixture was concentrated under a reduced pressure to fractionate ethanol, and an oily substance in the residue was separated and refined in a silica gel column chromatography by use of a developing solvent (hexane:ethyl acetate = 2:1), thereby obtaining 4-(m-nitrophenyl)-2-dimethoxymethyl-6-methyl-1,4-dihydropyridine-3,5-dicarboxylic acid 3-β-propoxyethyl ester-5-ethyl ester (4.5 g.) in the form of light yellow oily substance.

UV (in MeOH): $\lambda_{max}$ = 225, 355 nm.

NMR (90 MHz, in DMSO-$d_6$): δ0.8 (t, J=8Hz, 3H), 1.18 (t, J=7Hz, 3H), 1.45 (m, 2H), 2.35 (s, 3H), 3.3 (t, J=8Hz, 2H), 3.4 (s, 6H), 3.52 (t, J=4Hz, 2H), 4.1 (q, J=7Hz, 2H), 4.12 (t, J=4Hz, 2H), 5.03 (s, 1H), 5.95 (s, 1H), 7.35—8.3 (m, 4H), 8.74 (s, 1H).

(b) 4-(m-Nitrophenyl)-2-dimethoxymethyl-6-methyl-1,4-dihydropyridine-3,5-dicarboxylic acid 3-β-propoxyethyl ester-5-ethyl ester (4.4 g.) was dissolved in acetone (50 ml), to which 6N hydrochloric acid (10 ml) was added. The mixture was reacted for three hours under agitation at a room temperature. After the reaction mixture was neutralized with sodium hydrogen carbonate, it was concentrated under a reduced pressure to extract residue with ethyl acetate. The extracted solution was rinsed with water and dried, followed by concentration under a reduced pressure to distill off the solvent. Then, the residue was separated and refined in a silica gel column chromatography using a developer solvent (hexane:ethyl acetate = 3:1), thereby obtaining 4-(m-nitrophenyl)-2-formyl-6-methyl-1,4-dihydropyridine-3,5-dicarboxylic acid 3-β-propoxyethyl ester-5-ethyl ester (3.2 g.) in the form of light yellow oily substance.

UV (in MeOH): $\lambda_{max}$ = 235, 355 nm.

NMR (90 MHz, in DMSO-$d_6$): δ0.85 (t, J=8Hz, 3H), 1.19 (t, J=7Hz, 3H), 1.55 (m, 2H), 2.45 (s, 3H), 3.4 (t, J=7Hz, 2H), 3.6 (t, J=4Hz, 2H), 4.1 (q, J=7Hz, 2H), 4.15 (t, J=4Hz, 2H), 5.08 (s, 1H), 7.4—8.3 (m, 4H), 9.05 (s, 1H).

(c) 4-(m-Nitrophenyl)-2-formyl-6-methyl-1,4-dihydropyridine-3,5-dicarboxylic acid 3-β-propoxyethyl ester-5-ethyl ester (3.1 g.) was dissolved in ethanol (50 ml), to which sodium borohydride (0.6 g.) was added. The reaction was conducted for one hour at a temperature of from 0 to 10°C. The reaction mixture solution was adjusted to a pH value of 4 (1N—HCl), after which it was concentrated under a reduced pressure. The residue was extracted with ethyl acetate. The extracted solution was washed with water, dried, and concentrated under a reduced pressure. The residue was refined in a silica gel column chromatography using a developer solvent (hexane:ethyl acetate = 1:1) to thereby obtain an oily substance (2.9 g.). This oily substance was crystallized from ethyl acetate-hexane to obtain 4-(m-nitrophenyl)-2-hydroxymethyl-6-methyl-1,4-dihydropyridine-3,5-dicarboxylic acid 3-β-propoxyethyl ester-5-ethyl ester (2.3 g.) in crystalline form.

mp: 111 to 119°C.

UV (in MeOH): $\lambda_{max}$ = 236, 355 nm

IR (KBr): 3430, 3340, 2960, 1690, 1665, 1635, 1590, 1530, 1470, 1350, 1310, 1270, 1200, 1110, 1100, 900, 830, 780, 760, 710 $cm^{-1}$.

NMR (90 MHz, in DMSO-$d_6$): δ0.87 (t, J=8Hz, 3H), 1.52 (m, 2H), 2.45 (s, 3H), 3.38 (t, J=8Hz, 2H), 3.58 (t, J=5Hz, 2H), 4.05 (q, J=8H, 2H), 4.15 (t, J=5Hz, 2H), 4.72 (d, J=5Hz, 2H), 5.12 (s, 1H), 5.67 (t, J=5Hz, 1H), 7.5—8.3 (m, 4H), 8.7 (s, 1H).

Invention

Example 1

(a) 2-(m-Nitrobenzylidene)-4,4-dimethoxyacetoacetic acid β-propoxyethyl ester (4 g.) and 3-amino crotonic acid β-propoxyethyl ester (2 g.) were dissolved in propanol (30 ml), and the reaction, treatments, and separation and refining were conducted in the same manner as in comparative Example 1 (a) above, whereby 4-(m-nitrophenyl)-2-dimethoxymethyl-6-methyl-1,4-dihydropyridine-3,5-dicarboxylic acid bis(β-propoxyethyl ester) (3.7 g.) in light yellow oily substance was obtained.

UV (in MeOH): $\lambda_{max}$ = 225, 355 nm.

NMR (90 MHz, in DMSO-$d_6$): δ0.8 (t, J=7Hz, 6H), 1.47 (m, 4H), 2.35 (s, 3H), 3.29 (t, J=7Hz, 4H), 3.45 (s, 6H), 3.53 (t, J=4Hz, 4H), 4.08 (t, J=4Hz, 4H), 5.02 (s, 1H), 5.95 (s, 1H), 7.3—8.3 (m, 4H), 8.74 (s, 1H).

(b) Using 4-(m-nitrophenyl)-2-dimethoxymethyl-6-methyl-1,4-dihydropyridine-3,5-dicarboxylic acid bis(β-propoxyethyl ester) (3.6 g.), the reaction, treatments, and separation and refining were carried out in the same manner as in Comparative Example 1 (b) above, thereby obtaining 4-(m-nitrophenyl)-2-formyl-6-methyl-1,4-dihydropyridine-3,5-dicarboxylic acid bis(β-propoxyethyl ester) (3.1 g.) in light yellow oily substance.

UV (in MeOH): $\lambda_{max}$ = 235, 350 nm.

NMR (20 MHz, in DMSO-$d_6$): δ0.95 (t, J=7Hz, 6H), 1.6 (m, 4H), 2.45 (s, 3H), 3.4 (t, J=7Hz, 4H), 3.55 (t, J=5Hz, 4H), 4.2 (t, J=5Hz, 4H), 5.08 (s, 1H), 7.4—8.3 (m, 4H), 9.05 (s, 1H).

(c) Using 4-(m-nitrophenyl)-2-formyl-6-methyl-1,4-dihydropyridine-3,5-dicarboxylic acid bis(β-propoxyethyl ester) (3 g.), the reaction, treatments, and separation and refining were done in the same manner as in Comparative Example 1 (c) above, thereby obtaining crystalline 4-(m-nitrophenyl)-2-hydroxymethyl-6-methyl-1,4-dihydropyridine-3,5-dicarboxylic acid bis(β-propoxyethyl ester) (2 g.).

mp: 64—71 (from diisopropylether-hexane).

UV (in MeOH): $\lambda_{max}$ = 237, 355 nm.

IR (KBr): 3400, 3340, 2970, 2880, 1700, 1670, 1640, 1590, 1530, 1470, 1350, 1205, 1100, 1065, 900, 830, 780, 760, 710 $cm^{-1}$.

NMR (90 MHz, in DMSO-$d_6$): δ0.95 (t, J=7Hz, 6H), 1.5 (m, 4H), 2.42 (s, 3H), 3.33 (t, J=7Hz, 4H), 3.52 (t, J=5Hz, 4H), 4.1 (t, J=5Hz, 4H), 4.63 (d, J=4Hz, 2H), 5.03 (s, 1H), 5.56 (t, J=4Hz, 1H), 7.4—8.3 (m, 4H), 8.58 (s, 1H).

Example 2

(a) 2-(m-Nitrobenzylidene)-4,4-dimethoxyacetoacetic acid β-propoxyethyl ester (4 g.) and 3-amino crotonic acid β-aryloxyethyl ester (1.7 g.) were dissolved in propanol (30 ml), and the reaction, treatments, and separation and refining were done in the same manner as in Comparative Example 1 (a) above, thereby obtaining oily 4-(m-nitrophenyl)-2-dimethoxymethyl-6-methyl-1,4-dihydropyridine-3,5-dicarboxylic acid 3-β-propoxyethyl ester-5-β-allyloxyethyl ester (4.6 g.).

(b) Using 4-(m-nitrophenyl)-2-dimethoxymethyl-6-methyl-1,4-dihydropyridine-3,5-dicarboxylic acid 3-β-propoxyethyl ester-5-β-aryloxyethyl ester (4.5 g.), the reaction, treatments, and separation and refining were done in the same manner as in Comparative Example 1 (b) above, thereby obtaining oily 4-(m-nitrophenyl)-2-formyl-6-methyl-1,4-dihydropyridine-3,5-dicarboxylic acid 3-β-propoxyethyl ester-5-β-allyloxyethyl ester (3.8 g.).

(c) Using 4-(m-nitrophenyl)-2-formyl-6-methyl-1,4-dihydropyridine-3,5-dicarboxylic acid 3-β-propoxyethyl ester-5-β-aryloxyethyl ester (3.5 g.), there were conducted the same reaction, treatments, and separation and refining as in Comparative Example 1 (c) above, thereby obtaining powdery 4-(m-nitrophenyl)-2-hydroxymethyl-6-methyl-1,4-dihydropyridine-3,5-dicarboxylic acid 3-β-propoxyethyl ester-5-β-allyloxyethyl ester (1.2 g.).

UV (in MeOH): $\lambda_{max}$ = 236, 355 nm.

IR (KBr): 3450, 3380, 2950, 2870, 1685, 1650, 1600, 1530, 1470, 1350, 1270, 1200, 1100, 1000, 920, 900, 825, 780, 740, 705 $cm^{-1}$.

NMR (90 MHz, in DMSO-$d_6$): δ0.81 (t, J=7Hz, 3H), 1.45 (m, 2H), 2.38 (s, 3H), 3.28 (t, J=7Hz, 2H), 3.52 (t, J=4Hz, 4H), 3.9 (d, J=6Hz, 2H), 4.08 (t, J=4Hz, 4H), 4.6 (d, J=5Hz, 2H), 5.02 (s, 1H), 5.0—5.35 (m, 2H), 5.55 (t, J=5Hz, 1H), 5.4—6.0 (m, 1H), 7.35—8.2 (m, 4H), 8.57 (s, 1H).

Example 3 (intermediate)

(a) 2-(m-Nitrobenzidine)-acetoacetic acid β-chloroethyl ester (3.6 g.) and 4,4-dimethoxy-3-amino crotonic acid β-propoxyethyl ester (2.5 g.) were dissolved in propanol (30 ml), and the same reaction and treatments as in Comparative Example 1 (a) above were carried out to obtain oily 4-(m-nitrophenyl)-2-dimethoxymethyl-6-methyl-1,4-dihydropyridine-3,5-dicarboxylic acid 3-β-propoxyethyl ester-5-β-chloroethyl ester (5.1 g.).

(b) Using 4-(m-nitrophenyl)-2-dimethoxymethyl-6-methyl-1,4-dihydropyridine-3,5-dicarboxylic acid 3-β-propoxyethyl ester-5-β-chloroethyl ester (5 g.), the reaction and treatments were conducted in the same manner as in Comparative Example 1 (b) above, thereby obtaining oily 4-(m-nitrophenyl)-2-formyl-6-methyl-1,4-dihydropyridine-3,5-dicarboxylic acid 3-β-propoxyethyl ester-5-β-chloroethyl ester (4.1 g.).

(c) Using 4-(m-nitrophenyl)-2-formyl-6-methyl-1,4-dihydropyridine-3,5-dicarboxylic acid 3-β-propoxyethyl ester-5-β-chloroethyl ester (4 g.), the same reaction, treatments, and separation and refining as in Comparative Example 1 (c) above were conducted, whereby crystalline 4-(m-nitrophenyl)-2-hydroxymethyl-6-methyl-1,4-dihydropyridine-3,5-dicarboxylic acid 3-β-propoxyethyl ester-5-β-chloroethyl ester (2.5 g.) was obtained.

UV (in MeOH): $\lambda_{max}$ = 235, 355 nm.

IR (KBr): 3450, 3380, 2950, 1685, 1600, 1530, 1470, 1350, 1210, 1100, 900, 830, 805, 780, 740, 705 $cm^{-1}$.

NMR (90 MHz, in DMSO-$d_6$): δ0.83 (t, J=7Hz, 3H), 1.5 (m, 2H), 2.4 (s, 3H), 3.3 (t, J=7Hz, 2H), 3.77 (t, J=4Hz, 2H), 4.25 t, J=4Hz, 2H), 4.62 (s, 2H), 5.03 (s, 1H), 5.6 (br.s, 1H), 7.4—8.2 (m, 4H), 8.64 (s, 1H).

Example 4

4-(m-Nitrophenyl)-2-hydroxymethyl-6-methyl-1,4-dihydropyridine-3,5-dicarboxylic acid 3-β-propoxyethyl ester-5-β-chloroethyl ester (2.4 g.) obtained in Example 3 (c) above was dissolved in propanol (100 ml). To this solution, N-methyl-N-benzylamine (3 ml) and sodium iodide (0.1 g.) were added, and the reaction was continued for four hours, while refluxing. The reaction mixture was then concentrated under a reduced pressure, and the residue was extracted with ethyl acetate. The extracted solution was washed twice with 10% sodium hydrogen carbonate solution (10 ml), further washed with water, and dried, after which it was concentrated under a reduced pressure. The residue was refined in a silica gel column chromatography using ethyl acetate as a developer solvent. The sedimented substance was collected from aceton and hydrochloric acid ethanol, which was recrystallized from aceton-ethyl acetate to obtain crystalline 4-(m-nitrophenyl)-2-hydroxymethyl-6-methyl-1,4-dihydropyridine-3,5-dicarboxylic acid 3-β-propoxyethyl ester-5-β-(N-methyl-N-benzylamino)ethyl ester hydrochloric acid salt (0.88 g.).

UV (in MeOH): $\lambda_{max}$ = 235, 355 nm.

IR (KBr): 3420, 3280, 2960, 1680, 1530, 1505, 1350, 1250, 1195, 1100, 1030, 830, 780, 740, 700 $cm^{-1}$.

NMR (90 MHz, in DMSO-$d_6$): δ0.8 (t, J=7Hz, 3H), 1.45 (m, 2H), 2.4 (s, 3H), 2.6 (s, 3H), 3.3 (t, J=7Hz, 2H), 3.52 (t, J=3Hz, 2H), 3.7 (t, J=4Hz, 2H), 4.1 (t, J=3Hz, 2H), 4.25 (s, 2H), 4.45 (t, J=4Hz, 2H), 4.7 (s, 2H), 5.03 (s, 1H), 5.6 (br.s, 1H), 7.3—8.2 (m, 9H), 9.2 (s, 1H).

Comparative Example 2

(a) 2-(m-Nitrobenzylidene)-4,4-dimethoxyacetoacetic acid β-propoxyethyl ester (3.81 g.) and 4,4-dimethoxy-3-amino crotonic acid ethyl ester (1.9 g.) were dissolved in propanol (30 ml). The solution was then subjected to the reaction, treatments, and separation and refining in the same manner as in Comparative Example 1 (a) above, thereby obtaining oily 4-(m-nitrophenyl)-2,6-bis(dimethoxymethyl)-1,4-dihydropyridine-3,5-dicarboxylic acid 3-ethyl ester-5-β-propoxyethyl ester (4.7 g.).

UV (in MeOH): $\lambda_{max}$ = 270 nm

IR (KBr): 3410, 2950, 1725, 1690, 1615, 1530, 1475, 1350, 1275, 1210, 1190, 1100, 990, 905, 810, 740, 690 $cm^{-1}$.

NMR (90 MHz, DMSO-$d_6$): δ0.85 (t, J=8HGz, 3H), 1.17 (t, J=Hz, 3H), 1.48 (m, 2H), 3.27 (t, J=8Hz, 2H), 3.32 (s, 6H), 3.42 (s, 6H), 3.6 (t, J=4Hz, 2H), 3.8—4.3 (m, 4H), 5.1 (s, 1H), 5.7—6.0 (m, 2H), 7.4—8.3 (m, 5H).

(b) Using 4-(m-nitrophenyl)-2,6-bis(dimethoxymethyl)-1,4-dihydropyridine-3,5-dicarboxylic acid 3-ethylester-5-β-propoxyethylester (4.5 g.), the reaction, treatments, and separation and refining were conducted in the same manner as in Comparative Example 1 (b) above, thereby obtaining oily 4-(m-nitrophenyl) - 2,6 - diformyl - 1,4 - dihydropyridine - 3,5 - dicarboxylic acid 3-ethylester - 5 - β - propoxyethylester (3.5 g.).

UV (in MeOH): $\lambda_{max}$ = 236, 360 nm.

IR (KBr): 3400, 2980, 2950, 1730, 1700, 1610, 1530, 1480, 1350, 1280, 1210, 1095, 1070, 990, 910, 810, 775, 740, 690 $cm^{-1}$.

NMR (90 MHz, in DMSO-$d_6$): δ0.83 (t, J=7Hz, 3H), 1.18 (t, J=7Hz, 3H), 1.48 (m, 2H), 3.32 (t, J=7Hz, 2H), 3.62 (t, J=4Hz, 2H), 3.8—4.3 (m, 4H), 5.1 (s, 1H), 7.4—8.3 (m, 4H).

(c) Using 4-(m-nitrophenyl)-2,6-diformyl-1,4-dihydropyridine-3,5-dicarboxylic acid 3-ethylester-5-β-propoxyethyl ester (3.4 g.), the reaction, treatments, and separation and refining were done in the same manner as in Comparative Example 1 (c) above, whereby 4-(m-nitrophenyl)-2,6-bis(hydroxymethyl)-1,4-dihydropyridine-3,5-dicarboxylic acid 3-ethylester-5-β-propoxyethyl ester (1.3 g.) was obtained.

UV (in MeOH): $\lambda_{max}$ = 235, 356 nm.

IR (KBr): 3450, 3270, 2950, 1720, 1675, 1605, 1530, 1500, 1350, 1260, 1205, 1190, 1100, 1070, 1020, 980, 905, 830, 760, 740, 690 $cm^{-1}$.

NMR (90 MHz, in DMSO-$d_6$): δ0.84 (t, J=7Hz, 3H), 1.15 (t, J=7Hz, 3H), 1.48 (m, 2H), 3.3 (t, J=7Hz, 2H), 3.57 (t, J=4Hz, 2H), 4.02 (q, J=7Hz, 2H), 4.15 (t, J=4Hz, 2H), 4.76 (d, J=6Hz, 4H), 5.1 (s, 1H), 6.02 (t, J=6Hz, 2H), 7.5—8.3 (m, 4H), 9.25 (s, 1H).

**Claims**

1. A 1,4-dihydropyridine derivative which is 4-(m-nitrophenyl)-2-hydroxymethyl-6-methyl-1,4-dihydropyridine-3,5-dicarboxylic acid 3-β-propoxyethyl ester -5-β-allyloxyethylester.

2. A 1,4-dihydropyridine derivative which is 4-(m-nitrophenyl)-2-hydroxymethyl-6-methyl-2,4-dihydropyridine-3,5-dicarboxylic acid bis(β-propoxyethyl ester).

3. A 1,4-dihydropyridine derivative which is 4-(m-nitrophenyl)-2-hydroxymethyl-6-methyl-1,4-dihydropyridine-3,5-dicarboxylic acid 3-β-propoxyethyl ester-5-β(N-methyl-N-benzylamino)-ethyl ester hydrochloric acid salt.

4. A process for preparing 1,4-dihydropyridine derivatives represented by the following general formula (I) and the pharmacologically acceptable salts thereof

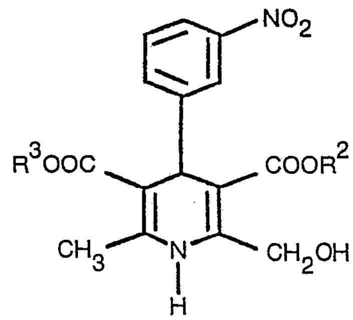

(I)

where $R^2$ is β-propoxyethyl and $R^3$ is β-propoxyethyl β-allyloxyethyl, or β-(N-methyl-N-benzylamino)-ethyl, said process comprising:

a) reacting a compound represented by the general formula (II) with an amine compound represented by the general formula (III), or reacting a compound represented by the general formula (IV) with an amine compound represented by the general formula (V) to prepare a compound represented by the formula (VI):

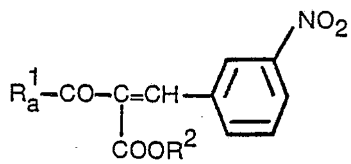

(II)

where $R_a^1$ represents dialkoxymethyl and $R^2$ is β-propoxyethyl

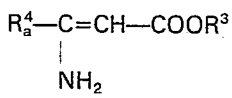

(III)

where $R^3$ represents ß-propoxyethyl, ß-allyloxyethyl or ß-(N-methyl-N-benzylamino)ethyl and $R_a^4$ denotes methyl;

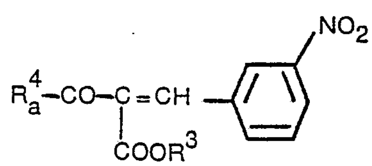

(IV)

where $R^3$ and $R_a^4$ represent the same groups as mentioned in the general formula (III);

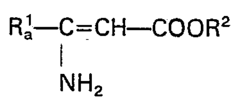

(V)

where $R_a^1$ and $R^2$ represent the same groups as mentioned in the general formula (II);

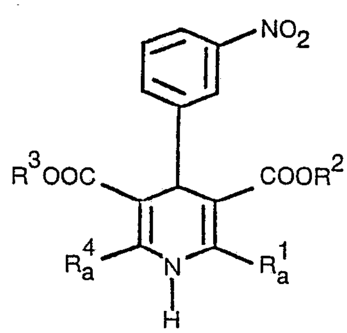

(VI)

where $R_a^1$, $R^2$, $R^3$ and $R_a^4$ represent the same groups as mentioned in the preceding general formulas (II) to (V);

(b) hydrolyzing the compound represented by the general formula (VI) to prepare a compound represented by the general formula (VII):

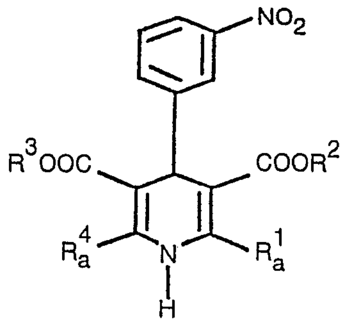

(VI)

where $R_a^1$, $R^2$, $R^3$ and $R_a^4$ represent the same groups as mentioned in the preceding general formulas (II) to (V);

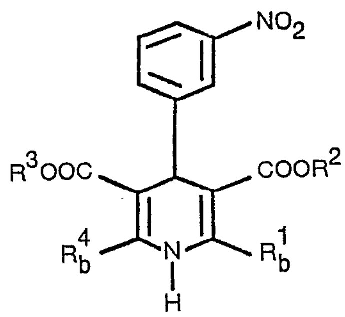

(VII)

where $R_b^1$ represents formyl; $R_b^4$ denotes methyl and $R^2$ and $R^3$ indicate the same groups as mentioned in the foregoing general formulas;

(c) reducing the compound of the general formula (VII) to prepare a compound represented by the general formula (VIII):

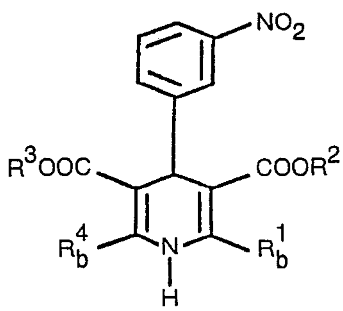

(VII)

where $R_b^1$ represents formyl; $R_b^4$ denotes methyl; and $R^2$ and $R^3$ indicate the same groups as mentioned in the foregoing general formulas;

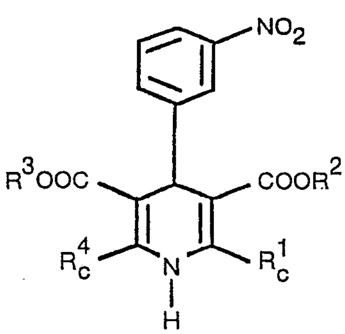

(VIII)

where $R_c^1$ denotes hydroxymethyl $R_c^4$ represents methyl; and $R^2$ and $R^3$ represent the same groups as mentioned in the foregoing general formulas; and

(d) reacting a compound represented by the general formula IX with N-methyl-N-benzylamine

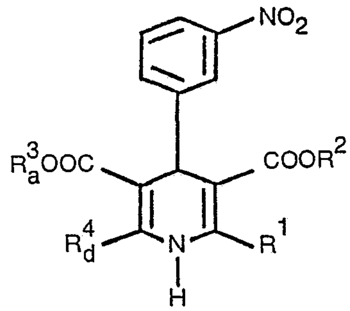

(IX)

where $R^1$ is hydroxymethyl, $R^3_a$ represents haloethyl; $R^4_d$ denotes methyl; and $R^2$ is the same group as in the preceding general formulas;
to prepare a compound represented by the general formula (XI):

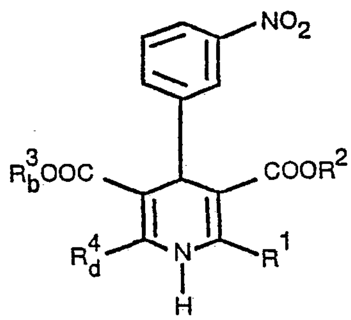

(XI)

where $R^3_b$ is β-(N-methyl-N-benzylamino)ethyl and $R^1$, $R^2$ and $R^4_d$ are the same groups as mentioned in the foregoing general formulas.

**Patentansprüche**

1. 1,4-Dihydropyridinderivat, nämlich 4(m-Nitrophenyl)-2-hydroxymethyl-6-methyl-1,4-dihydropyridin-3,5-dicarbonsäure-3-ß-propoxyethylester-5-ß-allyloxyethylester.

2. 1,4-Dihydropyridinderivat, nämlich 4(m-Nitrophenyl)-2-hydroxymethyl-6-methyl-2,4-dihydropyridin-3,5-dicarbonsäure-bis-(ß-propoxyethylester).

3. 1,4-Dihydropyridinderivat, nämlich 4(m-Nitrophenyl)-2-hydroxymethyl-6-methyl-1,4-dihydropyridin-3,5-dicarbonsäure-3-β-propoxyethylester-5-β-(N-methyl-N-benzylamino)-ethylesterchlorwasserstoffsäuresalz.

4. Verfahren zur Herstellung von 1,4-Dihydropyridinderivaten der folgenden allgemeinen Formel (I) sowie der pharmakologisch akzeptablen Salze derselben

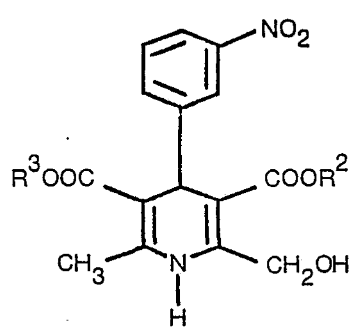

(I)

wobei $R^2$ für β-Propoxyethyl steht und
$R^3$ für β-Propoxyethyl β-Allyloxyethhyl oder β-(N-methyl-N-benzylamino)-ethyl steht, dadurch gekennzeichnet, daß man
(a) eine Verbindung der allgemeinen Formel (II) mit einer Aminverbindung der allgemeinen Formel (III) umsetzt oder eine Verbindung der allgemeinen Formel (IV) mit einer Aminverbindung der allgemeinen Formel (V) umsetzt, um eine Verbindung der allgemeinen Formel (VI) zu erhalten:

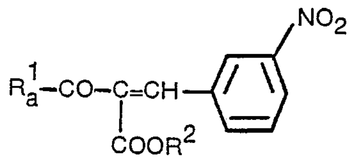

(II)

wobei $R_a^1$ für Dialkoxymethyl steht und $R^2$ für β-Propoxyethyl steht;

$$R_a^4—\underset{\displaystyle NH_2}{\underset{|}{C}}=CH—COOR^3$$ (III)

wobei $R^3$ für β-Propoxyethyl, β-Allyloxyethyl oder β-(N-Methyl-N-benzylamino)ethyl steht und $R_a^4$ Methyl bezeichnet;

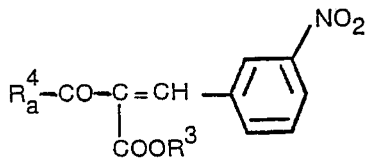

(IV)

wobei $R^3$ und $R_a^4$ die gleichen Gruppen bezeichnen, die im Zusammenhang mit der allgemeinen Formel (III) erwähnt wurden;

$$R_a^1—\underset{\displaystyle NH_2}{\underset{|}{C}}=CH—COOR^2$$ (V)

wobei $R_a^1$ und $R^2$ die gleichen Gruppen bedeuten, wie sie bei der allgemeinen Formel (II) erwähnt wurden;

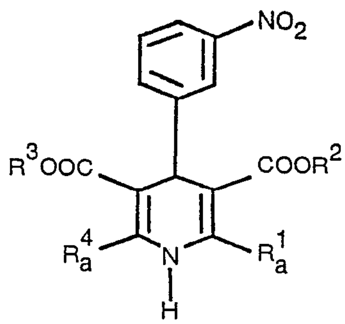

(VI)

wobei $R_a^1$, $R^2$, $R^3$ und $R_a^4$ die gleichen Gruppen bedeuten, wie sie bei den vorstehenden allgemeinen Formeln (II) bis (V) erwähnt wurden;

(b) die Verbindung der allgemeinen Formel (VI) hydrolysiert, um eine Verbindung der allgemeinen Formel (VII) zu erhalten

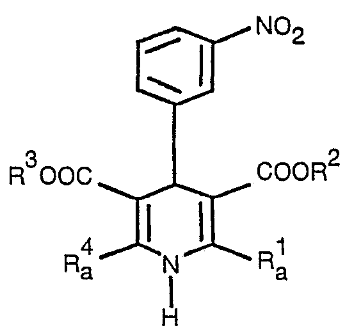

(VI)

wobei $R_a^1$, $R^2$, $R^3$ und $R_a^4$ die gleichen Gruppen bedeuten, wie sie bei den vorstehenden allgemeinen Formeln (II) bis (V) erwähnt wurden;

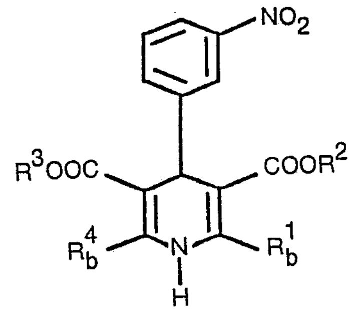

(VII)

wobei $R_b^1$ für Formyl steht; $R_b^4$ Methyl bedeutet und $R^2$ und $R^3$ die gleichen Gruppen bedeuten, wie sie bei den vorstehenden allgemeinen Formeln erwähnt wurden;

(c) die Verbindung der allgemeinen Formel (VII) reduziert, um eine Formel der allgemeinen Formel (VIII) herzustellen

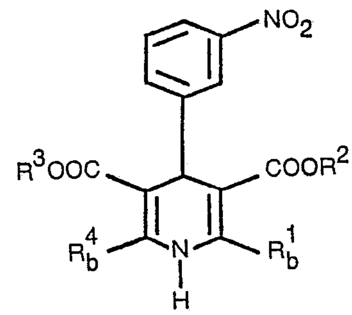

(VII)

wobei $R_b^1$ für Formyl steht; $R_b^4$ Methyl bezseichnet und $R^2$ und $R^3$ die gleichen Gruppen bedeuten, wie sie bei den vorstehenden allgemeinen Formeln erwähnt wurden;

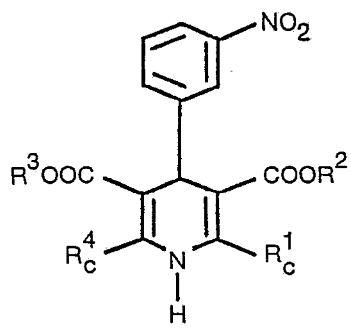

(VIII)

wobei $R_c^1$ für Hydroxymethyl steht; $R_c^4$ Methyl bedeutet und $R^2$ und $R^3$ die gleichen Gruppen bedeuten, wie sie bei den vorstehenden allgemeinen Formeln erwähnt wurden; und

(d) eine Verbindung der allgemeinen Formel (IX) mit N-Methyl-N-benzylamin umsetzt

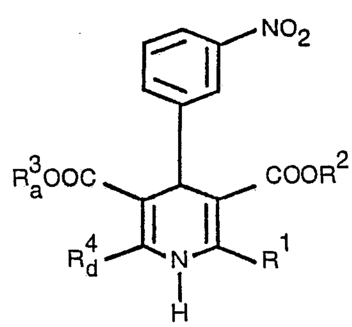

(IX)

wobei $R^1$ für Hydroxymethyl steht; $R_a^3$ Halogenethyl bedeutet; $R_c^4$ für Methyl steht; und $R^2$ die gleiche Gruppe bedeutet wie bei den vorstehenden allgemeinen Formeln;

um eine Verbindung der allgemeinen Formel (XI) zu erhalten

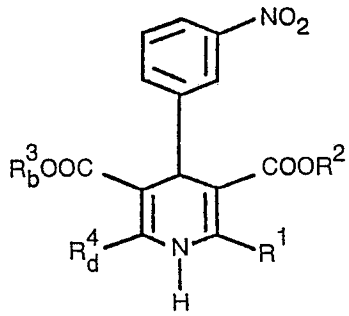

(XI)

wobei $R^3_b$ für β-(N-methyl-N-benzylamino)ethyl steht und $R^1$, $R^2$ und $R^4_d$ die gleichen Gruppen bedeuten, wie sie bei den vorstehenden allgemeinen Formeln erwähnt wurden.

**Revendications**

1. Un dérivé de la 1,4-dihydropyridine qui est le 4-(m-nitrophényl)-2-hydroxyméthyl-6-méthyl-1,4-dihydropyridine-3,5-acide dicarboxylique 3-β-propoxyéthyl ester-5-β-allyloxyéthyl ester.

2. Un dérivé de la 1,4-dihydropyridine qui est le 4-(m-nitrophényl)-2-hydroxyméthyl-6-méthyl-2,4-dihydropyridine-3,5-acide dicarboxylique bis (β-propoxyéthyl ester).

3. Un dérivé de la 1,4-dihydropyridine qui est le chlorhydrate du 4-(m-nitrophényl)-2-hydroxyméthyl-6-méthyl-1,4-dihydropyridine-3,5-acide dicarboxylique 3-ß-propoxyéthyl ester-5-ß-(N-méthyl-N-benzyl-amino)-éthyl ester.

4. Un procédé de préparation des dérivés de la 1,4-dihydropyridine représentés par la formule générale (I) suivante et de leurs sels pharmacologiquement acceptables

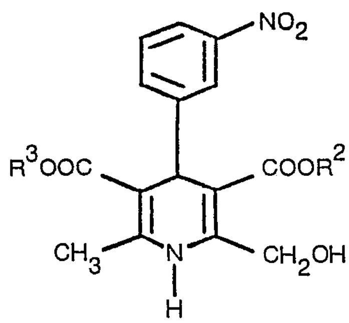

(I)

dans laquelle $R^2$ est le groupe β-propoxyéthyle et $R^3$ est le groupe β-propoxyéthyle, β-allyloxyéthyle ou β-(N-méthyl-N-benzylamino)-éthyle, ledit procédé comprenant:

a) la réaction d'un composé représenté par la formule générale (II) avec un composé du type amine représenté par la formule générale (III), ou la réaction d'un composé représenté par la formule générale (IV) avec un composé du type amine représenté par la formule générale (V) pour préparer un composé représenté par la formule (VI):

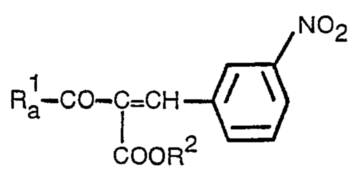

(II)

dans laquelle $R^1_a$ représente un groupe dialcoxyméthyle et $R^2$ est le groupe β-propoxyéthyle;

$R^4_a$—C=CH—COOR$^3$
| 
$NH_2$

(III)

dans laquelle $R^3$ représente le groupe β-propoxyéthyle, β-allyloxyéthyle ou β-(N-méthyl-N-benzylamino) éthyle et $R^4_a$ désigne le groupe méthyle;

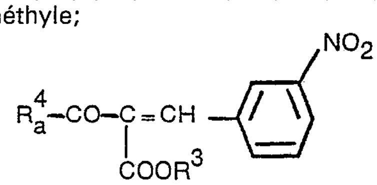

(IV)

dans laquelle $R^3$ et $R_a^4$ représentent les mêmes groupes que ceux mentionnés pour la formule générale (III);

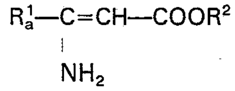

(V)

dans laquelle $R_a^1$ et $R^2$ représentent les mêmes groupes que ceux mentionnés pour la formule générale (II);

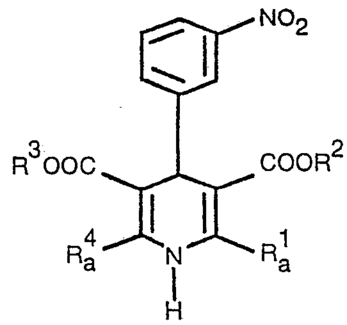

(VI)

dans laquelle $R_a^1$, $R^2$, $R^3$ et $R_a^4$ représentent les mêmes groupes que ceux mentionnés pourles formules générales (II) à (V) précédentes;

(b) l'hydrolyse du composé représenté par la formule générale (VI) pour préparer un composé représenté par le formule générale (VII):

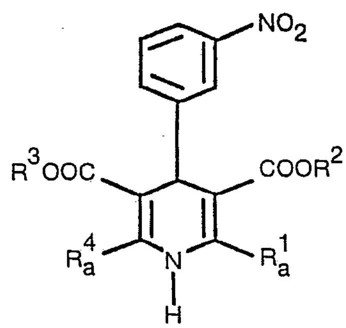

(VI)

dans laquelle $R_a^1$, $R^2$, $R^3$ et $R_b^4$ représentent les mêmes groupes que ceux mentionnés pour les formules générales (II) à (V) précédentes;

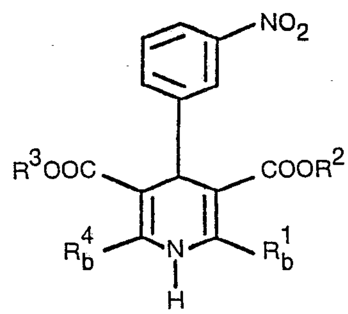

(VII)

dans laquelle $R_b^1$ représente le groupe formyle; $R_b^4$ désigne le groupe méthyle et $R^2$ et $R^3$ désignent les mêmes groupes que ceux mentionnés pour les formules générales précédentes;

(c) la réduction du composé de formule générale (VII) pour préparer un composé représenté par la formule générale (VIII):

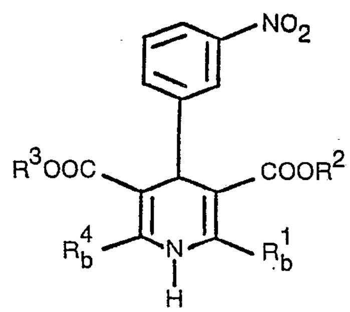

(VII)

dans laquelle $R_b^1$ représente le groupe formyle; $R_b^4$ désigne le groupe méthyle; et $R^2$ et $R^3$ représentent les mêmes groupes que ceux mentionnés pour les formules générales précédentes;

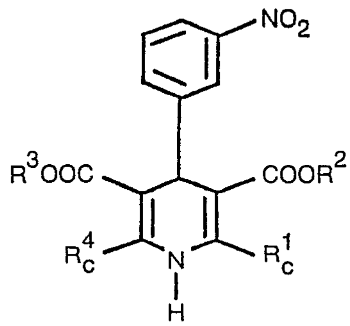

(VIII)

dans laquelle $R_c^1$ désigne le groupe hydroxyméthyle; $R_c^4$ représente le groupe méthyle; et $R^2$ et $R^3$ représentent les mêmes groupes que ceux mentionnés pour les formules générales précédentes; et

(d) la réaction d'un composé représenté par la formule générale (IX) avec la N-méthyl-N-benzylamine

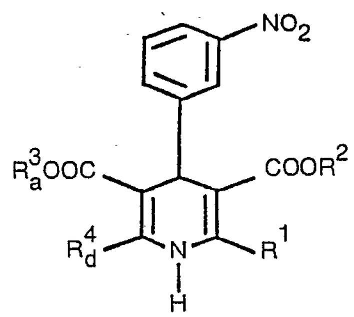

(IX)

dans laquelle $R^1$ est le groupe hydroxyméthyle, $R_a^3$ représente un groupe haloéthyle; $R_d^4$ désigne le groupe méthyle; et $R^2$ est le même groupe que dans les formules générales précédentes;

pour préparer un composé représenté par la formule générale (XI):

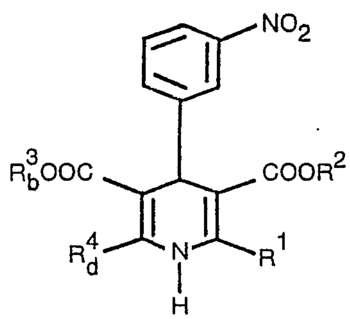

(XI)

dans laquelle $R_b^3$ représente le β-(N-méthyl-N-benzylamino) éthyle et $R^1$, $R^2$, et $R_d^4$ sont les mêmes groupes que ceux mentionnés pour les formules générales précédentes.